# EUROPEAN PATENT APPLICATION

(11) **EP 1 900 359 A1**
(43) Date of publication of application: **19.03.2008**
(21) Application number: 06019423.0
(22) Date of filing: 16.09.2006
(51) Int. Cl.: A61K 9/20

(54) **Oral modified release formulations containing drospirenon and 8-prenylnaringenin for use in female contraception**

(71) Applicant: KAIROSmed GmbH, 10117 Berlin (DE)
(72) Inventor: Hümpel, Michael, Dr., 14163 Berlin (DE); Schleuning, Wolf-Dieter, Dr., 13467 Berlin (DE); Heuermann, Arno, Dipl.-Ing., 10115 Berlin (DE); Krings, Matthias, Dr., 80992 München (DE); Thunecke, Markus, Dr., 14193 Berlin (DE)
(74) Representative: Jungblut, Bernhard Jakob

(57) **Abstract**

This invention is directed to an oral modified release formulation of the phytoestrogen 8-Prenylnaringenin in combination with a progestin, preferably the progestin Drospirenone, and its use for combination oral contraception.

## Description

This invention is related to oral modified release formulations containing a progestin, preferably the synthetic progestin Drospirenon (DRSP), and the estrogenic natural compound 8-Prenylnaringenin (8-PN) and their use in female contraception.

Combination oral contraceptives (COC) consisting of a progestin and an estrogen have been known since the early 1960's. Numerous analogues have been developed since the first so-called one-phase preparations consisting of a fixed daily dose of a progestin and an estrogen for 21 days followed by a 7 days drug-free interval. Innovations have mainly been aimed at the reduction of drug-free intervals, different absolute dose levels of active ingredients, the progestin/estrogen dose ratios during one treatment cycle, and the choice of the progestin. Starting with the 19-nor-testosterone derivative Norethisterone (NET) many pro-drugs were developed which either in part or completely are converted into NET (NET-AC, lynestrenol, norethinodrel) *in vivo.* One pharmacological property of NET, its prodrugs and other 19-nor-testosterone derivatives or their active metabolites (e.g. desogestrel, norgestimate, norgestrel, levonorgestrel) is their partially androgenic activity leading to unwanted effects among which sebaceous skin and body weight gain are the most common ones. Other progestins have been derived from progesterone (the so-called C-21 progestins). Medroxyprogesterone acetate (MPA), chlormadinone acetate (CMA), and cyproterone acetate (CPA) are the best known representatives of this chemical class. Like natural progesterone these compounds do not activate the androgen receptor and consequently do not exhibit androgenic activity. In contrast, MPA, CMA, and - more pronounced - CPA show a partial anti-androgenic activity which can be used for the treatment of acne and other symptoms caused by enhanced testosterone levels in women. However, all these progestins lack the anti-mineral-corticoid activity of progesterone. Recently such a progestin was found as a progestagenic metabolite of a spironolactone derivative. Drospirenone (DRSP) has been
characterized as a highly potent progestin with some anti-androgenic and aldosterone antagonistic activities and thus almost perfectly mimicks the pharmacological profile of natural progesterone. The structure of DRSP is as follows:

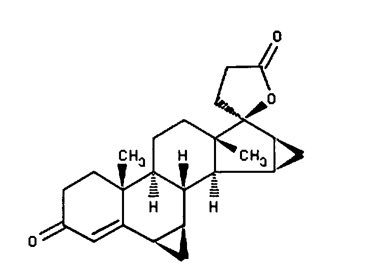

The aldosterone-antagonistic activity of DRSP were the subject of a patent application in 1976 (Schering AG). Another patent application was filed in 1977 claiming the substance as a diuretic drug. Its use as a progestin in oral contraception and gynecological indications was patented in 1980. Thus, the patent protection of the substance ran out at the same time when the drug came to the market as constituent of the combined oral contraceptive Yasmin® (introduction into the market in Germany was November 2000). The patent protection of Yasmin® is mainly based on a technical patent claiming a stable oral formulation with acute drug release (US 6,787,531).
DRSP 3 mg in combination with 0.03 mg EE has been on the market as Yasmin® since 2000 (Germany) or 2001 (USA). Treatment schedule is 21 days followed by a 7 days drug-free interval . Just recently, the EE reduced version YAZ® (DRSP 3 mg plus EE 0.02 mg taken for 24 days followed by a 4 days drug-free interval) was granted FDA approval.
In contrast to the large variety of progestins used in COCs, their estrogenic component almost exclusively consists of ethinyl estradiol (EE) or its pro-drug mestranol. EE is a synthetic derivative of the most potent natural estrogen, 17ß-estradiol (E2). By 17α-ethinylation the rate of metabolic degradation of E2 in the liver is slowed down leading to an increase of oral bioavailability of E2 from roughly 1 - 3% of dose to about 40 % of dose (EE). However, with respect to oral bioavailability large variations exist between individuals. The coefficient of variation (CV) of the mean area under the plasma level time curve (AUC) after oral 30 µg EE ranges between 75 and 94 %. Compared to DRSP (CV of AUC's of about 20 %) this figure shows that the systemic availability of the estrogenic component of COCs is at much higher variance than that of the progestogenic component. This might be the main reason why very often an original COC starts with a higher EE dose (mostly 0.03 mg/d) but is then followed by an EE reduced version (mostly 0.02 mg/d). Although the pharmacokinetics of EE do not meet the requirements for a reliable support of an highly active drug there were only a few attempts to substitute EE in COCs. One refers to the use of E2, but even high doses of 4mg/d or more did not resolve the problem of insufficient bleeding control in respective developmental COCs. This can be explained by the fact that the product of concentration and time at the estrogen receptor (ER) was not sufficiently high for E2 because very rapid metabolic inactivation of E2 is an integral part of its biological regulation process. Most probably, E2 concentrations at the ER were below a threshold level for many hours of the 24 hours treatment interval.
In summary, there is a medical need for a substitution of EE as the estrogen in COCs. The substitute should reliably (CV of AUC < 50 %) become bioavailable after oral administration and be present at sufficiently high concentrations at the ER for the total treatment interval of 24 hours.
Interestingly, COCs are always preparations with acute drug release. This is surprising because it is well known that the dose of drugs with high oral bioavailability can be reduced by modified release administration. As an example, the oral dose of levonorgestrel as so-called minipill (Microlut®) is 0.03 mg/d (continuous treatment; Pearl Index = 1). An even increased clinical effect (Pearl Index = 0.05-0.1 can be reached with 0.02 mg/d released from an intrauterine device by zero order kinetics (Mirena®). A non-hormonal IUD has a Pearl Index of about 2.
Thus, a progestin dose sparing effect of about 30 % can be realized by a modified release formulation of COCs. However, the throughout used estrogen component in COCs is not a suitable drug for such a preparation because the first liver pass metabolism would degrade EE to a higher extent than after acute release. In consequence, to reach the same biological effect higher EE doses would become necessary in modified release formulations and the degree of variability of systemic EE availability would not decrease but rather further increase.
Recently, a new estrogen, 8-Prenylnaringenin (8-PN), identified in plants (hop, *Anaxagorea luzonensis* A. Gray) was shown to be not only the most active phyto-estrogen but also a substance that exhibits tissue specificity with a much lower activity on the uterus than E2 at equivalent bone protective doses. 8-PN (5,7-Dihydroxy-2-(4-hydroxyphenyl)-8-(3-methylbut-2-enyl)-chroman-4-on) has the structure given below.
The 4-hydroxyphenyl group may either be in the 2S(-) or the 2R (+) position. 8-PN was characterized as a pure estrogen devoid of binding or activation of the PR, AR, or GR and without any anti-estogenic activity. 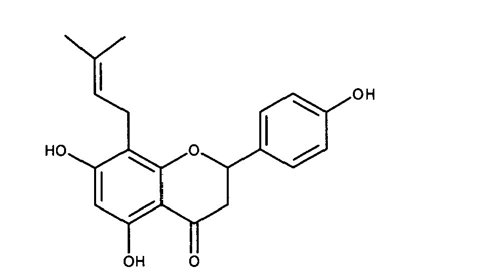 Interestingly, 8-PN is mainly inactivated by conjugation. 8-PN conjugates are excreted predominantly with the bile fluid, deconjugated in the duodenum and intestinum after bile fluid secretion and then re-absorbed as unchanged drug. This process is known as entero-hepatic re-circulation (EHC) and is the basis for a multiple re-use of drug in question. 8-PN exhibits a stable systemic availability with a CV of 42 or 29 % of mean AUC at oral doses of 250 or 750 mg, respectively. In addition, due to a surprisingly low extent of metabolism by CYP isoenzymes 8-PN shows a stable systemic availability irrespective of the mode of administration, i.e. acute or modified release. Thus, 8-PN is a suitable drug for use in modified release formulations and can overcome the barrier posed by the PK of EE to develop COCs as modified release formulations.

The normal route of administration in COC is the oral one. This route is not only preferred because of user convenience but also because most of the substances in question need a high daily dose which is difficult to achieve otherwise such as the nasal, dermal or inhalatory routes. Just recently a combination transdermal system for hormonal contraception was developed (Ortho Evra®) and approved by the FDA. Ortho Evra® continuously releases 0.15 mg norelgestromin and 0.02 mg EE per day and is characterized by a Pearl Index of 1. The lower contraceptive efficacy as compared to Ortho Cyclen® (0.25 mg norgestimate plus 0.035 mg EE per unit) and the higher estrogenic efficacy as compared to the oral formulation is surprising at the first glance. However, norelgestromin is one active metabolite of norgestimate and norgestrel is another highly active metabolite. By surpassing the first liver pass metabolism (patch) the extent of metabolic generation of norgestrel is decreased and overall progestogenic activity also decreased. On the other hand, calculating with a mean oral bioavailability of 40 % of oral EE dose only 0.014 mg EE is the equivalent after dermal absorption. Thus, by different reasons the Ortho Evra® system shows a lower efficacy (Pearl Index) and an increased (generally accepted as health risk) estrogenic activity as compared to the oral preparation. This example clearly shows that the oral route of administration is not only more convenient but also safer than the dermal route. This is especially true when high drug fluctuations after oral administration can be avoided by a modified release system and thereby incorporate the advantage of a quasi constant drug influx as is demonstrated for the dermal application.

The problem, therefore, is to provide an oral formulation for a suitable so-called C21-progestin, preferably DRSP, and a substitute for EE (mestranol), preferably 8-PN, which continuously distributes the drugs for the gastro-intestinal transit time of generally 12-16 hours and which preferably has to be administered once a day, preferably in the evening.
This problem was solved by a solid oral modified release formulation containing 8-PN and DRSP. This formulation contains a polymeric matrix, a buffer substance and one or more excipients in addition to 8-PN and DRSP. Preferably, the buffer substance is an alkaline substance like e. g. magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof. Preferably, the particle size of the compounds is in the range of 0.1 - 750 µm. Most preferably, it is in the range of 20 - 400 µm. The polymer matrix is preferably chosen from the group consisting of: cellulose derivatives, acrylic derivatives, vinyl polymers, polyacrylates, polycarbonates, polyethers, polystyrenes polyanhydrides, polyesters, polyorthoesters, polysaccharides and natural polymers. Most preferably, the polymer matrix consists of water soluble polyvinylpyrrolidone and/or water insoluble polyvinylacetate. In a preferred embodiment the oral modified release formulation is coated with a polymeric coat.

Excipients may be chosen from the group consisting of lactose, calcium phosphate, manitol and starch. Preferably, an additional excipient is microcrystalline cellulose.

The solid oral modified release formulations of this invention show a pH-independent drug release in vitro. This is important as the pH varies considerably in the gastro-intestinal tract and continuous release should be achieved independent of the pH. In contrast to C21-progestins (MPA, CPA, DRSP), however, the solubility of 8-PN is pH-dependent. The compound shows higher solubility at higher pH-values whereas the solubility of the compound is low at lower pH-values. The low acid solubility property of 8-Prenylnaringenin results in vitro in slow drug dissolution at pH 1, whereas the dissolution is fast at higher pH-values such as pH 6.8. The resulting dissolution profiles are different at different pH-values. This problem is solved by the solid oral modified release formulation of this invention. In addition it is known that DRSP is sensitive to low pH-values at which the carbolactone ring can be opened to the inactive acid. It was also shown that ring opening does not occur at the pH of the stomach (no loss in bioavailability) but low pH-values have to be avoided during the manufacturing process. This problem is solved by the solid oral modified release formulation of this invention.

The solid oral modified release formulation of this invention solves the problem of a continuous distribution of 8-PN almost over 24 h. This is a combined effect of the pharmacokinetic profile of 8-Prenylnaringenin (high metabolic stability versus CYP isoenzymes, EHC) which is drastically different from those of other estrogens and the solid oral modified release formulation. The solid oral modified release formulation of this invention also solves the problem of a dose sparing effect of C21-progestins, preferably DRSP, by continuous distribution of the drug for 12 - 16 hours and increased drug trough levels at steady state conditions.

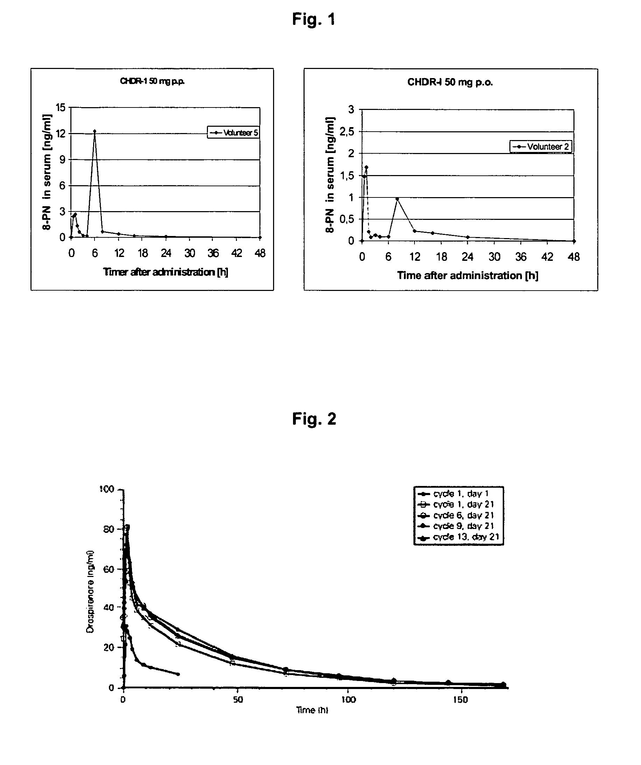

The pharmacokinetic profile of 8-PN is characterized by a complete oral absorption, a low degree of metabolisation (less than 60 % of dose) and a high pre-systemic elimination (first-liver-pass excretion; about 55 % of dose). The high and unexpected pre-systemic elimination leads to a collection of substantial dose parts in the bile fluid from which these dose parts are secreted into the duodenum when gastric signaling occurs with a meal uptake. Respective dose parts are then absorbed again and reach systemic circulation. Examples for this effect (drug serum levels) are shown in Fig. 1. Data were taken from the clinical Phase la study and represent two volunteers of the lowest dose group (50 mg 8-Prenylnaringenin).

Taking the total area under the curve as a measure for 8-Prenylnaringenin systemic availability, the percentage of the area under the second peak (generated by reabsorption of the pre-systemically eliminated and then biliary secreted dose parts) can be used to estimate those dose parts which undergo pre-systemic elimination after oral dosing. On an average of six women investigated this figure is 54 ± 20 %.

The invention is to combine this unexpected pharmacokinetic property of 8-Prenylnaringenin with a modified release formulation, which can release the drug at an almost constant rate for 8 - 10 hours. The drug release from the solid oral modified release formulation according to this invention is preferably close to or perfect zero order kinetics. This oral modified release formulation is preferably taken in the evening (after dinner or before bed-time). During nighttime 8-PN is released at almost constant rate leading to flat drug serum levels and a collection of substantial dose parts in the bile fluid which - after reabsorption - account for more than half of total systemically available dose. The next day these dose parts are secreted into the duodenum when the first meal (breakfast or lunch) is taken. Re-absorption gives rise to elevated 8-Prenylnaringenin serum levels over the first half of the day, which - at a lower level - repeats with dinner in the evening. Overall, the total systemically available dose is distributed over 24 hours avoiding unnecessary high peaks and ensuring effective drug concentrations in target tissues. The anticipated daily dose is between 50 and 250 mg, preferably between 75 and 150 mg. The oral modified release formulation is preferably taken 6 -12 hours, more preferably 8 - 12 hours before having a meal.

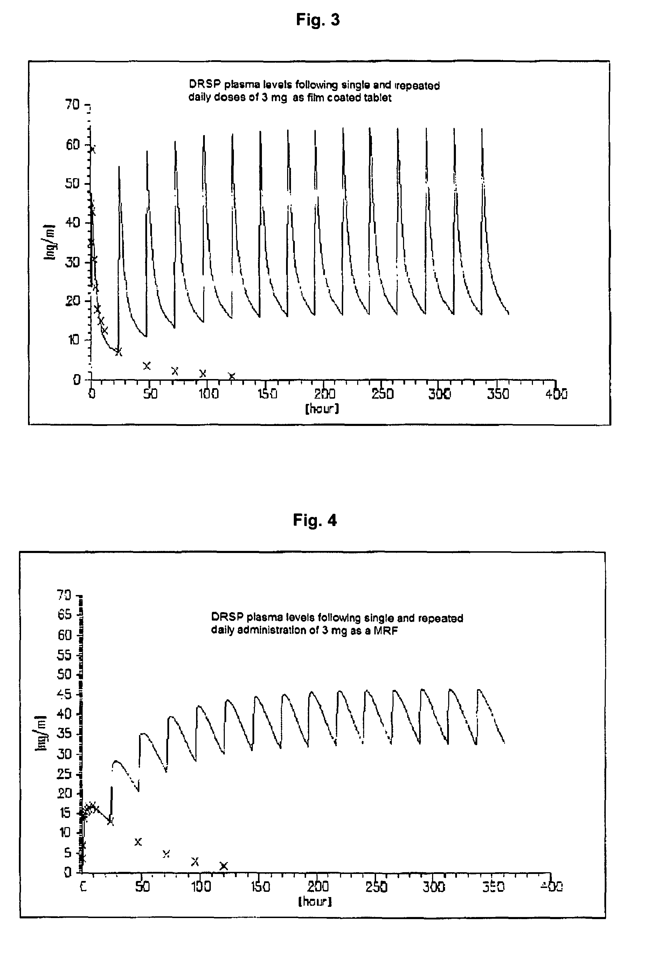

The pharmacokinetic profile of DRSP is characterized by complete oral absorption, an absolute oral bioavailability of 76 %, and a disposition half-life of 31 hours. The apparent volume of distribution was estimated to 4 Ukg. After oral intake of 3 mg DRSP maximum serum levels of 37 ng/ml were reached at 1.7 hours. AUC₀₋₂₄ₕ was 288 ngxh/ml. Following daily repeated administration the maximum serum level increased to about 80 ng/ml and AUC to about 920 ngxh/ml. Increases are the result of drug accumulation at daily treatment and a half-life of 31 hours. Figure 2 shows time course and degree of accumulation.
DRSP does not bind to SHBG and does not suppress EE induced SHBG or CBG serum levels. DRSP is only slightly metabolized, mainly by CYP3A4.
DRSP shows a 2 to 3 fold accumulation after acute drug release from the film-coated tablet. The accumulation factor (AF) of the simulated drug serum levels is 2.4 in Figure 3. The simulated mean steady state through level was 17 ng/ml. Following repeated oral administration of a MRF a similar AF was found (AF=2.5 in Figure 4) but higher through levels (32 ng/ml) and much lower fluctuations of DRSP serum levels were found (Fig.4). Higher through level after repeated administration of the MRF let suggest a decrease of daily doses by at least 30 %.

This invention provides a method of hormonal contraception by administering a solid oral modified release formulation containing 8-PN and DRSP to the patient once daily.

Still another aspect, this invention provides a method of maintaining therapeutically effective levels of 8-PN and DRSP in human plasma by administering an 8-PN and DRSP containing oral modified release formulation once daily.

The method includes administering an oral modified release formulation including from about 5 to about 80 % by weight 8-PN and less than 5 % by weight DRSP in one dosage form per dose to women, to maintain 8-PN plasma levels from about 0.5 to about 5 ng 8-PN/ml and to maintain DRSP plasma levels from about 15 to about 80 ng DRSP/ml for at least 24 hours wherein the dose is administered once a day.

The formulations of this invention may be either in the form of single unit dosages, e.g. tablets, or in the form of multiple unit dosages, e.g. granulates, pellets or mini-tablets. These multiple unit dosages may be filled in gelatine capsules or compressed to tablets.

The single unit dosages may be produced by powder blending and direct compression into tablets or by powder blending, granulation and compression into tablets. The tablets may be coated by a film.

Multiple unit dosage may be produced by extrusion/spheronization, by layering technique, by rotor granulation, by powder blending and direct compression into mini tablets, by powder blending, granulation and compression into mini tablets, by powder blending, direct compression into mini tablets and film coating or by powder blending, granulation, compression into mini tablets and film coating. 8-Prenylnaringenin can be produced by the method described in WO 2005/037816. Drospirenone can be produced by the method described in US 6,933,395.

### Description of the figures

Fig. 1 shows drug serum levels following a single oral dose of 50 mg 8-Prenylnaringenin in two postmenopausal women; 8-Prenylnaringenin was administered as a 1:1 mixture with lactose (gelatine capsule) at fasted state in the morning, lunch was served 6 hours later. Re-increases in drug serum levels show re-absorption of dose parts collected in the bile fluid and subsequently secreted triggered by lunch.
Fig. 2 shows DRSP serum levels after oral administration of 3 mg DRSP combined with 0.03 mg EE in 13 women. The COC was taken for 13 cycles at a 21 days/7 days regimen. Samples were drawn on day 1 of cycle 1 and on day 21 of cycles 1, 6, 9, and 13. The figure was taken from Blode H, Wuttke W, Loock W, Heithecker R (2000): The European Journal of Contraception and Reproductive Care, 5; 256-264
Fig 3 shows mean (N=13) DRSP serum levels following a single oral administration of 3 mg as a rapidly drug releasing film coated tablet and a simulation of daily repteated administrations; single dose data were generated from day 21, cycle 1 (Blode et al, 2000) by subtraction of prevalue or figures resulting from a prevalue decrease with a half-life of 30 hours. Simulation was performed using the software TopFit 2.0.
Fig. 4 shows the DRSP serum levels following a single oral dose of 3 mg as modified release formulation and a simulation of daily repeated administrations of a MRF; single dose data were generated by a simulation of a zero order release of drug during the first 10 hours after ingestion and a drug level decrease with a half-life of about 30 hours thereafter. Basic PK data for simulation were taken from: Blode H, Wuttke W, Loock W, Heithecker R (2000): The European Journal of Contraception and Reproductive Care, 5; 256-264

### Examples:

### Example 1

Preparation of a modified release formulation containing 8-Prenylnaringenin and Drospirenone

### Production of Mini-Matrix Tablets by Means of Direct Tableting

| | |
|---|---|
| 2.333 mg | of 8-Prenylnaringenin |
| 0.046 mg | Drospirenone |
| 1.000 mg | of Kollidon SR^{®} |
| 1.946 mg | of lactose |
| 1.500 mg | microcrystalline cellulose |
| 0.070 mg | of highly dispersed silicon dioxide |
| 0.105 mg | of magnesium stearate |

8-Prenylnaringenin, DRSP, Kollidon SR^{®}, lactose and microcrystalline cellulose are sieved individually and mixed in a turbula mixer for 10 minutes. Highly dispersed silicon dioxide, sieved; is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 2

### Production of Mini-Matrix Tablets by Means of Direct Tableting

| | |
|---|---|
| 2.333 mg | of 8-Prenylnaringenin |
| 0.046 mg | Drospirenone |
| 1.000 mg | of Kollidon SR^{®} |
| 1.169 mg | of magnesium oxide |
| 0.777 mg | of lactose |
| 1.500 mg | microcrystalline cellulose |
| 0.070 mg | of highly dispersed silicon dioxide |
| 0.105 mg | of magnesium stearate |

8-Prenylnaringenin, DRSP, Kollidon SR^{®}, magnesium oxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 3

### Production of Mini-Matrix Tablets by Means of Direct Tableting

| | |
|---|---|
| 2.333 mg | of 8-Prenylnaringenin |
| 0.046 mg | Drospirenone |
| 1.000 mg | of Kollidon SR^{®} |
| 1.169 mg | of magnesium hydroxide |
| 0.777 mg | of lactose |
| 1.500 mg | microcrystalline cellulose |
| 0.070 mg | of highly dispersed silicon dioxide |
| 0.105 mg | of magnesium stearate |

8-Prenylnaringenin, DRSP, Kollidon SR^{®}, magnesium hydroxide, lactose and microcrystalline cellulose are sieved individually and mixed in a turbula mixer for 10 minutes. Highly dispersed silicon dioxide, sieved, is added, and all components are mixed in the turbula for another 5 minutes. Magnesium stearate, sieved, is spread on, and all components are mixed in the turbula for another 30 seconds. Tableting of the powder mixture into mini-matrix tablets is carried out by means of an eccentric tablet press or a rotary tablet press.
The release from these mini-tablets is measured by means of the method that is mentioned in Example 4.

### Example 4

### Measurement of the Release of 8-PN and DRSP

Measurement of the active ingredients release from mini-matrix tablets is carried out according to a one-compartment method (basket apparatus), as described in U.S. Pharmacopeia USP XXV. The release of 8-PN and DRSP is examined in phosphate buffer solution, pH 6.8 (composition, see USP XXV). Ten percent (w/w) hydroxypropyl-ß-cyclodextrine is added in order to achieve sink conditions and primarily control the drug release by the dosage form.

## Claims

1. Oral modified release formulation containing 8-Prenylnaringenin, Drospirenone, a polymeric matrix, a buffer substance and one or more excipients.

2. Oral modified release formulation containing 8-Prenylnaringenin, Drospirenone a polymeric matrix, a buffer substance and one or more excipients and where the particle size of the compounds is in the range of 0.1 - 750 µm.

3. Oral modified release formulation containing 8-Prenylnaringenin and Drospirenone according to claim 1 or 2 wherein the buffer substance is an alkaline substance.

4. Oral modified release formulation containing 8-Prenylnaringenin and Drospirenone according to claims 1, 2 or 3 wherein said formulation is coated with a polymeric coat that affects the dissolution of active ingredients.

5. Oral modified release formulation according to claims 1 - 4 wherein the polymer matrix is chosen from the group of the following materials: cellulose derivatives, acrylic derivatives, vinyl polymers, polyacrylates, polycarbonates, polyethers, polystyrenes polyanhydrides, polyesters, polyorthoesters, polysaccharides and natural polymers.

6. Oral modified release formulation according to claims 1 - 4 wherein the polymer matrix is chosen from water soluble polyvinylpyrrolidone and water insoluble polyvinylacetate.

7. Oral modified release formulation according to claims 1 - 6 wherein the buffer substance is magnesium oxide, magnesium hydroxide, dihydroxyaluminum aminoacetate, magnesium carbonate, calcium carbonate, sodium ascorbate, magnesium trisilicate, dihydroxyaluminum sodium carbonate, aluminum hydroxide, sodium citrate, potassium phosphate, sodium bicarbonate, disodium hydrogenphosphate or some combinations thereof.

8. Oral modified release formulation according to claims 1 - 7 wherein the formulation contains an additional lubricant.

9. Oral modified release formulation according to claims 1 - 8 wherein the excipient is lactose, calcium phosphate, manitol or starch.

10. Oral modified release formulation according to claims 1-9 wherein the formulation contains microcrystalline cellulose as an additional excipient.

11. Oral modified release formulation according to claims 1 - 10 wherein the formulation contains silicon dioxide as flow promoter.

12. Oral modified release formulation according to claims 1-11 wherein the particle size of the powder mixtures is between 20 - 400 µm.

13. Use of the oral modified release formulation according to claims 1-12 for the production of a medicament for the combination hormonal contraception.

14. Use of the oral modified release formulation according to claims 1-13 where the preferred daytime of ingestion is the evening or bedtime.

15. Use of the oral modified release formulation according to claims 1-14 where the active treatment phase is between 21 and 25 days.

16. Use of the oral modified release formulation according to claims 1-15 where 8-Prenylnaringenin is replaced by any other estrogenic compound or any derivative of 8-Prenylnaringenin.

17. Use of the oral modified release formulation according to claims 1-15 where Drospirenone is replaced by any other progestin.
